## Europäisches Patentamt
## European Patent Office
## Office européen des brevets

(11) Publication number: **0 025 699**
**B1**

(12) **EUROPEAN PATENT SPECIFICATION**

(45) Date of publication of patent specification: **12.10.83**

(51) Int. Cl.³: **A 61 D 7/00, A 61 M 31/00**

(21) Application number: **80303201.0**

(22) Date of filing: **11.09.80**

(54) Device for drug delivery to ruminants.

(30) Priority: **12.09.79 US 74683**

(43) Date of publication of application:
**25.03.81 Bulletin 81/12**

(45) Publication of the grant of the patent:
**12.10.83 Bulletin 83/41**

(84) Designated Contracting States:
**DE GB IT LU NL SE**

(56) References cited:
**DE - A - 2 824 288
FR - A - 2 287 242
GB - A - 1 316 998
US - A - 3 710 795
US - A - 3 832 252
US - A - 3 854 480
US - A - 3 920 805
US - A - 3 926 188**

(73) Proprietor: **ELI LILLY AND COMPANY
307, East McCarty Street
Indianapolis Indiana 46285 (US)**

(72) Inventor: **Simpson, Barbara Ellen
7401 East 71st Street
Indianapolis Indiana 46256 (US)**

(74) Representative: **Crowther, Terence Roger
European Patent Attorney et al,
Erl Wood Manor
Windlesham Surrey GU20 6PH (GB)**

Courier Press, Leamington Spa, England.

# 0 025 699

## Device for drug delivery to ruminants

This invention relates to a novel and economical drug delivery device for releasing to the stomach of a ruminant a predetermined amount of pharmacological agent at a controlled and continuous rate for a prolonged period of time. The device is a metal cylinder designed to be charged with a suitable controlled release formulation, said charged device to be orally administered to a ruminant and retained in the reticulo-rumen portion of the ruminant stomach.

There currently are available several chemical compounds which exhibit a beneficial effect upon ruminant animals. For example, several compounds are known which improve feed efficiency when administered to ruminants. Other compounds are effective in promoting growth and improving the yield of meat utilized for human consumption. Still other compounds are effective in the therapeutic treatment and prophylactic control of ruminant diseases such as parasitic infestation. Many of these useful agents are effective only when administered via the rumen of the animal. Consequently, such drugs routinely are administered by daily dosing in the form of feed additives, licks, water additives and the like. Because some animals are range feb over a rather long period of time, it is impossible to administer drugs which require daily administration or delivery via feed additives or the like. Moreover, daily administration of drugs by injection to feed lot animals is uneconomical.

Slow release formulations designed to release an effective amount of active ingredient over a prolonged period of time are known in the art. However, many problems have been encountered in the development of effective means for economically administering such controlled release formulations to the rumen of domesticated ruminant animals. This is due to a number of factors. If the delivery device is too small in size and weight, it will not remain in the rumen sufficiently long to be effective, nor will it contain a sufficient quantity of active ingredient to achieve the desired sustained release effect. If the device is too large, administration to the animal is difficult or impossible. Moreover, foreign devices placed in the rumen are prone to expulsion by regurgitation or by passage into the intestine. Most important, however, is the fact that the delivery device must not permit excessive amounts of formulated drug to be administered to the animal, since excessive doses of certain drugs may be lethal.

A number of ruminant devices have been designed in an effort to overcome these problems. Laby, in U.S. Patent No. 3,844,285, describes an expandable device which allegedly is orally administered and then once in the rumen is adapted to physical change into an altered configuration which will prevent or at least hinder regurgitation of the device. Similarly, a spring-loaded device is described by Laby in German published specification 2,822,832. Such device is equipped with expandable plastic strips which are said to be effective in retaining the device in the rumen following oral administration. Marston, in U.S. Patent No. 3,056,724, describes a hollow pellet or shell device.

The prior art devices suffer from several disadvantages. In general, the variable geometry devices described by Laby are limited in use by the particular formulation. Additionally, such devices being made of plastic, are subject to destruction when an animal bites the device prior to swallowing, thereby permitting the release of what may be lethal doses of active ingredient. The Marston device has no mention of a coating present on the inner surface when used as a cylinder device, and such a device could release a lethal dose of active ingredient. Additionally, such devices are subject to mechanical failure, and none of the prior art devices are readily reusable, thus adding further to the expense involved in food production.

The present invention relates to a device for delivering controlled release formulations to the reticulo-rumen of ruminants so that a uniform and constant dosing of drug is realized which device is not subject to destruction during or after administration. The device of this invention is economically advantageous since it is inexpensive to manufacture and can be easily recovered from ruminants at the time of slaughter, and thus can be used repeatedly.

This invention concerns a novel drug delivery device for all delivery to the reticulo-rumen of a ruminant a controlled release formulation, said device comprising a steel cylinder open at both ends, said cylinder having an inside diameter to length ratio of 0.50 to 0.75, preferably of 0.55 to 0.75, having a density from 2.0 to 3.5 g/cc, formulation retaining means being provided on or in engagement with the inner wall of the cylinder, said device being coated with a substance which does not promote depolymerization of a copolymer derived from 60 to 95 weight percent lactic acid and 40 to 5 weight percent glycolic acid.

The invention provides a metal cylinder which is open at both ends, said cylinder being equipped with a retaining means within the inner part of the cylinder to facilitate retaining the formulated pharmacological agent within the cylinder. The device provides a constant surface area of exposed formulation so that uniform and constant delivery of drug is achieved. The device of this invention is designed for convenient oral administration to a ruminant, and once administered, the device is of such weight that it remains positioned in the reticulo-rumen portion of the ruminant stomach. Upon total pay-out of the controlled release formulation, the empty device remains in the reticulo-rumen until the animal is slaughtered, at which time it can be removed if desired and is capable of re-use.

In accordance with the present invention, there is provided a delivery device for use in the controlled treatment of ruminants utilizing sustained release formulations of pharmacological agents.

2

The device provided by this invention is a metal cylinder which is filled with a controlled release formulation and placed in the reticulo-rumen portion of the ruminant stomach. The animal then receives a constant and controlled dosage of active ingredient over a prolonged period of time by the biodegradation and erosion of the formulation support with concomitant uniform release of the active ingredient.

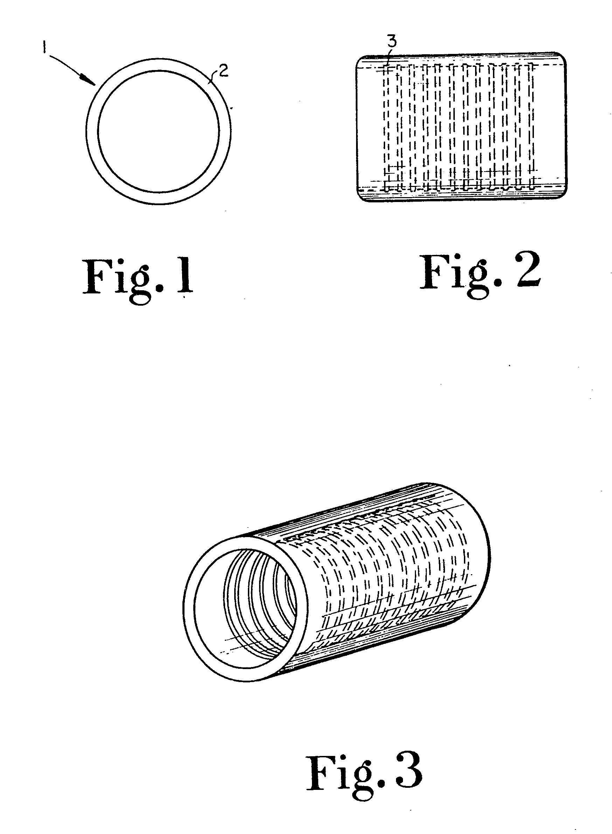

As illustrated in Fig. 1, the novel and useful drug delivery device 1 of this invention is a cylinder open at both ends. The device is made of steel, and preferably is manufactured from low carbon steel, or alternatively from stainless steel. The device, when made of low carbon steel, is coated with a metal or alloy which does not promote degradation of a controlled release formulation excipient such as a polymeric matrix. Such polymeric matrix materials are subject to depolymerization, a process that may be catalyzed by contact with metal such as steel. Coating the cylinder, particularly the inner surface, with a metal such as nickel, magnesium, silver, or aluminum or other suitable metal, or with a food grade enamel or lacquer e.g. epoxyphenolic resins, or with a plastic, decreases or eliminates such depolymerization. The device provided by this invention preferably is a low carbon or mild steel cylinder which is coated with nickel plating.

The wall 2 of Fig. 1 of the present device can be about 1.0 to about 2.0 millimeters thick. It has been found that a wall thickness of about 1.5 mm provides a device which is economical to manufacture and allows convenient insertion of a retaining means within the inner surface of the wall.

The drug delivery device provided by this invention is suited to administration of controlled release formulations to ruminants such as cattle, sheep and goats. The device will of course vary in size depending upon the particular animal species to be treated. However, the ratio of inside diameter to length will remain constant for all such devices. Specifically, the device of this invention will have a ratio of inside diameter to length of 0.55 to 0.75, preferably 0.60 to 0.70. For example, a capsule device of this invention suited for administration to young feeder calves will measure about 11 to about 35 millimeters in diameter (i.d.) and about 20 to about 64 millimeters in length. A typical capsule will measure about 20 mm in diameter and about 33 mm in length. A preferred device for administration to calves is one measuring about 30 mm. in diameter and about 50 mm. in length. A device ideally suited to administration to sheep and goats is one measuring about 15 millimeters in diameter and about 25 millimeters in length.

The density of the device provided by this invention is such that the device is retained in the reticulo-rumen of a ruminant, not only when filled with formulation, but also following total discharge of the formulation. The density will be from 2.0 to 3.5 g/cc. A preferred device suitable for administration to cattle is one measuring about 35 mm in length, with a density of about 2.9 g/cc.

As hereinabove pointed out, the inner surface of the cylinder drug delivery device is equipped with a retaining means. Such retaining means is a system to aid retention of a formulated drug which is filled into the device. It is important that the drug core and the cylinder inner wall be such that rumen fluids do not flow freely throughout the inner length of the device, since such action tends to loosen the formulated drug to such an extent that it can be expelled from the capsule. If the formulated drug is permitted to become expelled from the capsule, the exposed surface area of the core plug becomes too great, such that excessive amounts of active drug are released to the animal. Such excessive doses can, in some cases, be lethal to the animal.

The retaining means contemplated herein can be, among other things, a series of circular grooves cut into the inner wall of the steel cylinder. Such grooves are filled with formulation during the packing or charging of the steel delivery device, and thereby operate to aid retention of the formulation in the cylinder. As illustrated in Fig. 2, such grooves 3 can, if desired, be evenly spaced, and can vary in number depending on the length of the cylinder device utilized. Neither of these aspects is, however, critical to the operation of the device. Generally, the inner grooves will be spaced about 1 to about 5 millimeters apart, and each such groove can be about 0.2 to about 2.0 millimetres in width. The grooves can be cut in any convenient shape, for instance as V-shaped threads or as U-shaped flat bottomed cuts. Fig. 3 illustrates the device of Fig. 2 turned 45° to show these inner grooves. The preferred capsule to be utilized for the administration of controlled release formulations to calves is a steel cylinder measuring about 50 millimeters in length, said capsule having about 10 to about 14 inner grooves measuring about 0.6 to about 1.0 millimeters in width and spaced about 2 to about 3 millimeters apart. The grooves can be a depth of about 20 to about 50 percent of the wall thickness.

Additional or alternative internal retaining means can be utilized in the inner portion of the capsule device. For example, wire mesh, metal screens, steel pins, or rods can be attached across the diameter of the inner portion of the cylinder or dimples can be made in the inner portion of the cylinder such that the controlled release formulation can be packed or molded around such retaining means, thus further aiding retention of the drug core for a prolonged period of time commensurate with the pay-out period of the controlled release formulation of active ingredient.

A controlled release formulation to be administered utilizing the delivery device of this invention is one comprised of a pharmacologically active agent uniformly admixed with a copolymeric matrix. The copolymeric matrix which is ideally suited to administration via the capsule device provided herein is one derived from the condensation of 60 to 95 weight percent of lactic acid and 40 to 5 weight percent of glycolic acid. Said copolymer has an inherent viscosity of about 0.08 to about 0.30 when measured

3

in chloroform, and a molecular weight of about 6000 to about 35000. Such copolymer is prepared by a process which permits the substantially total removal of polymerization catalyst, thereby permitting the total biodegradation of the copolymer to naturally occurring substances when exposed to the stomach fluids of a ruminant. This is an especially significant advance in the art since the bulk of animals to be treated utilizing such copolymeric matrix are grown for human food consumption. It is therefore important that no toxic polymerization catalysts remain in the animal tissue once the copolymeric matrix has biodegraded.

The copolymers which are ideally suited to formulation of active drugs to be delivered by the novel delivery device of this invention are prepared by condensation of lactic acid and glycolic acid in the presence of a readily removable polymerization catalyst. Such catalysts include strong acid ion-exchange resins in the form of beads or similarly hard structures which are easily removed by filtration or similar techniques. Particularly preferred polymerization catalysts include commercially available strong acid ion-exchange resins [such as Amberlite IR-118 (H), Dowex HCR-W (formerly Dowex 50W), Duolite C-20, Amberlyst 15, Dowex MSC-1, Duolite C-25D, Duolite ES-26 (Trade Marks) and related strong acid ion-exchange resins]. The catalyst is added to a mixture of 60 to 95 parts by weight of lactic acid and 40 to 5 parts by weight of glycolic acid. The amount of catalyst utilized is not critical to the polymerization, but typically is from about 0.01 to about 20.0 parts by weight relative to the total weight of combined lactic acid and glycolic acid. The polymerization generally is carried out in the absence of solvents; however, organic solvents such as dimethylsulfoxide or N,N-dimethylformamide can be utilized if desired. The polymerization reaction routinely is carried out in a reaction system equipped with a condensing system, thereby permitting the collection and removal of water that is formed, as well as facilitating the removal of any lactide and glycolide by-products that are formed. The polymerization reaction generally is conducted at an elevated temperature of about 100 to about 250°C., and at such temperature is usually substantially complete within about 48 to about 96 hours. Ideally, the reaction can be carried out under a reduced pressure, thereby further facilitating removal of water and by-products.

The copolymer thus formed is readily recovered by simply filtering the molten reaction mixture, for example through a wire screen, to remove substantially all of the strong acid ion-exchange polymerization catalyst. Alternatively, the reaction mixture can be cooled to room temperature and then dissolved in a suitable organic solvent such as dichloromethane or acetone and then filtered by normal means so as to remove the solvent-insoluble strong acid ion-exchange resin. The copolymer then is isolated by removal of the solvent from the filtrate, for instance by evaporation under reduced pressure. Further purification of the copolymer can be accomplished if desired by re-dissolving it in a suitable organic solvent and further filtration, including the use of standard filter aids if desired.

Such copolymers, while not amenable to exact structure elucidation, are characterized as having a molecular weight of about 6000 to about 35000, and ideally about 25000. The copolymers are unique in that they are classified as high molecular weight substances having an inherent viscosity from about 0.08 to about 0.30 when measured by standard techniques utilizing the Ubbelohde viscometer in which chloroform has an efflux time of about 51 seconds at 25°C. The inherent viscosity of the copolymers is determined by the following equations:

$$\eta r = t/t_o \qquad \eta inh = \frac{\ln \eta r}{C}$$

wherein:

$\eta r$ is relative viscosity;

$t_o$ is efflux time of solvent;

$t$ is efflux time of the solution;

$\eta inh$ is inherent viscosity;

C is concentration in grams per 100 ml. of solvent; and

ln is logarithm.

The copolymers thus formed are additionally unique in that they are capable of providing a controlled release of pharmaceutical agents heretofore unavailable in reminant fluids.

The formulations which can be administered via the delivery device comprehended by this invention comprise an effective amount of a pharmacologically active ingredient such as a growth promoting agent uniformly admixed and dispersed throughout the copolymeric matrix hereinabove described. The formulations contain about 20 to about 80 percent by weight of active ingredient, ideally 30 to 70 percent. The pharmacologically active agents which can be utilized in the formulations include those agents commonly employed in the promotion of growth and stimulation of feed utilization by ruminants. Commonly used active agents include monensin, narasin, lasalocid, salinomycin, apramycin, actaplanin, deshydroxymethyl monensin, nigericin, deshydroxymethyl nigericin, dianemycin, erythromycin, vancomycin, ristocetin, soimycin, thiostrepton, desoxynarasin and the like. It will be recognized that salts and esters of such compounds can also be used. A particularly preferred formulation to be administered via the delivery device according to this invention comprises as active growth promotant the antibiotic monensin (see U.S. Patent No. 3,839,557) in the form of a sodium salt. Such growth promotant is preferably admixed with a copolymer containing about 70 to about 80 percent lactic units and about 30 to about 20 percent glycolic units, said copolymer having an inherent viscosity of about 0.13 to about 0.23. The formulations can, if desired, contain more than one active ingredient, as well as any of a number of commonly utilized pharmaceutical diluents, excipients and carriers.

The formulations to be filled into the device of this invention can be prepared in any of a number of ways including dry mixing, spray drying and the like. A preferred method of preparation comprises dissolving a suitable amount of the aforementioned copolymer in a solubilizing organic solvent that is readily removed by evaporation, and then adding the desired amount of pharmacologically active agent, followed by removal of the organic solvent. For example, about 50 grams of a copolymer derived from about 80 weight percent of lactic acid and about 20 weight percent of glycolic acid, having an inherent viscosity of about 0.18, can be dissolved in about 200 to about 400 ml. of a suitable organic solvent such as dichloromethane, acetone, dimethyl ether, tetrahydrofuran, chloroform, or the like. A pharmacologically active growth promoting agent, such as monensin sodium, lasalocid or salinomycin, in the amount of about 50 g., is then added to the dissolved copolymer. The solution thus formed is stirred for uniform mixing and then the solvent is removed by evaporation, thus providing a uniformly mixed formulation of copolymer and active agent in a solid mass. The solid so formed can be granulated and the granulation can be compressed and fused into the device of this invention for convenient oral administration to a ruminant. For instance, the formulation, after being introduced into the delivery device, can be administered orally to a range fed calf for effective growth promotion and/or enhanced feed utilization over a prolonged period of time. Such treatment provides uniformly controlled release of growth promotant to the ruminant, such that the effective dose of active ingredient is safe for the animal. Said effective dose typically amounts to less than about 500 mg. per animal each day. Typical daily doses will be about 100 to about 300 mg. per animal. The novel delivery device affords treatment to the animal for as long as about 160 days.

The formulations to be utilized in the delivery device of the invention can alternatively be prepared by first mixing the suitable copolymer and active agent such as monensin in the powdered dry state in order to provide a uniform powdered mixture. The mixture is next heated to about 80 to about 100°C. and extruded, for instance through a standard Killion Extruder, thereby providing a softened uniform mass which can be filled directly into the steel delivery device of this invention.

The formulations to be filled into the device of this invention can contain, in addition to the copolymer matrix and the active ingredient, other substances commonly utilized in medicinal formulations. Diluents, carriers, binders, excipients and adjuvants routinely incorporated in such formulations include gum tragacanth, acacia, corn starch, gelatin, alginic acid, magnesium stearate, aluminum monostearate, span 80, tween 80, sorbitan monostearate, hexaglyceryldistearate, glyceryl-distearate, sucrose, lactose, methylparaben, propylparaben, bees wax, mannitol, propylene glycol, microcrystalline cellulose, calcium silicate, silica, polyvinylpyrrolidone, cocoa butter, polyoxyethylene sorbitan monolaurate, ethyl lactate, sorbitan trioleate, calcium stearate, talc, and the like.

The formulations to be administered via the delivery device of this invention can, if desired, contain more than one active ingredient. Certain growth promoters, for example, can be combined and administered together in a device of this invention in order to achieve a synergistic growth promotion effect. Additionally, numerous benzimidazole compounds, for example fenbendazole and the like, are known to be effective anthelmintic agents when administered to ruminants. Such agents can be formulated together with a growth promoter such as monensin and administered via the device of this invention for the effective control of internal parasites and concomitant growth promotion.

The formulations which are to be administered in the delivery device of this invention are useful when administered to a ruminant and retained in the reticulo-rumen portion of the stomach of such ruminant. In order to function as contemplated, the formulation to be administered is filled into the capsule which is then capable of being retained in the reticulo-rumen of the animal, and such device provides a constant exposed surface area of the formulation such that the rumen fluids contact the formulation so that the desired controlled and uniform release of active agent is achieved.

For administration to ruminants such as cattle, the steel device hereinabove described can be filled with about 35 to about 60 g. of a controlled release formulation of active ingredient. Such bolus then is ready for oral administration to a calf for the uniform release of active ingredient, for example growth promotor or feed efficiency enhancer, over a period of time of about 80 to about 160 days.

For ruminants such as sheep, a steel capsule about 10 mm. to about 20 mm. in diameter and about 20 to about 30 mm. in length, open at both ends, can be filled with a suitable formulation for the controlled release of the desired active ingredient such as a growth promoting agent. Such agents, for instance monensin, cause an increase in feed utilization and/or effect growth in sheep not only of actual weight, but also of their generation of fleece.

The controlled release of active agent from formulations filled into the delivery device according to this invention has been demonstrated in both *in vitro* and *in vivo* experiments. In a typical *in vitro* study, a steel delivery device measuring 25 mm. by 25 mm. was filled with 11.0 grams of a fifty percent by weight formulation of monensin in a copolymer matrix derived from about 80 percent lactic acid and about 20 percent glycolic acid, said copolymer having an inherent viscosity of about 0.18. The bolus thus prepared (total weight 39.25 g) was placed in a plastic bottle containing 200 ml. of artificial rumen fluid at pH 7.0, prepared according to the method of Cheng et al., Journal of Dairy Science, *38*, 1225 (1955). The plastic bottle also contained twelve stainless steel ball bearings, each measuring 9 mm. in diameter. The bottle was rotated continuously at 34 rpm at a constant temperature of 39°C. Such conditions simulate the movement and abrasive effects of feed in the rumen of an animal. At twenty-four hour intervals over an eleven day test period, the capsule was removed, dried and weighed. The aqueous solution was removed from the plastic bottle at each twenty-four hour interval and assayed for its monensin content by the colorimetric method of Golab et al., Journal A.O.A.C., *56*, 171 (1973). Fresh solution was placed in the bottle at each twenty-four hour interval.

The results of such *in vitro* experiment are given in Tables I and II below. Table I gives the daily weight reduction of the monensin bolus. Table II gives the quantity of monensin found each day in the synthetic rumen fluid.

## TABLE I
### Monensin *in vitro* capsule weight change

| | 0 | 1 | 2 | 3 | 4 | (days) 5 | 6 | 7 | 8 | 9 | 10 | 11 |
|---|---|---|---|---|---|---|---|---|---|---|---|---|
| Weight (grams) | 39.25 | 38.57 | 38.2 | 37.7 | 37.2 | 36.7 | 36.25 | 35.8 | 35.45 | 34.8 | 34.3 | 33.75 |
| Weight change per day (grams) | | −0.5 | −0.55 | −0.5 | −0.5 | −0.5 | −0.45 | −0.45 | −0.35 | −0.65 | −0.5 | −0.55 |
| Cumulative weight change (grams) | | −0.5 | −1.05 | −1.55 | −2.05 | −2.55 | −3.0 | −3.45 | −3.8 | −4.45 | −4.95 | −5.5 |

## TABLE II
### Daily monensin content of *in vitro* rumen fluid

| Day | 1 | 2 | 3 | 4 | 5 | 6 | 7 | 8 | 9 | 10 | 11 |
|---|---|---|---|---|---|---|---|---|---|---|---|
| mg. found | 215 | 228 | 231 | 210 | 208 | 181 | 247 | 151 | 270 | 257 | 178 |
| mg. theory | 250 | 275 | 250 | 250 | 250 | 225 | 225 | 175 | 325 | 250 | 275 |
| Percent of theory found | 86 | 83 | 92 | 84 | 83 | 80 | 110 | 86 | 83 | 103 | 65 |

0 025 699

# 0 025 699

The *in vitro* data presented in Tables I and II demonstrate that in a simulated rumen environment, a controlled release formulation in a delivery device of this invention is effective in delivering a controlled and substantially uniform daily dose of active ingredient over an extended period of time.

The delivery devices provided by this invention additionally have been evaluated in *in vivo* systems. In one such study, mature cattle were equipped with a fistula for ready access to the reticulo-rumen portion of the stomach. Preweighed steel boluses, containing a formulation consisting of about 50 percent by weight of monensin sodium salt and 50 percent by weight of a copolymer derived from about 80 percent lactic acid and about 20 percent glycolic acid, were placed, via the fistula, into the rumen of each of three heifers. The animals were permitted to graze as desired, and were allowed to drink water freely. The formulation filled delivery devices were removed from the animals, via the fistula, at 7 to 13 day intervals over about a three month test period. Each device was weighed to determine the amount of active ingredient which had been administered to each animal, and then each device was returned to the reticulo-rumen via the fistula. The payout of active ingredient to each of the three test animals is given in Table III.

TABLE III

Payout of monensin from formulations packed in steel capsules
placed in the rumen of fistulated cattle

|  | Animal #1 | Animal #2 | Animal #3 |
|---|---|---|---|
| Gross bolus weight | 142.8 g | 142.3 g | 143.3 g |
| Empty bolus weight | 98.8 g | 97.9 g | 99.0 g |
| Net formulation weight (50% monensin) | 44.0 g | 44.4 g | 44.3 g |

| Payout periods (days) | Estimated Monensin payout mg/head/day (weight loss of the bolus divided by 2) | | |
|---|---|---|---|
| 0—7 | 0 | 96 | 100 |
| 7—14 | 164 | — | 221 |
| 14—21 | 243 | 193 | 150 |
| 21—29 | 194 | 200 | 206 |
| 29—42 | 227 | 192 | 246 |
| 42—52 | 175 | 185 | 170 |
| 52—63 | 182 | 195 | 227 |
| 63—72 | — | 183 | 239 |
| 73—79 | — | 71 | 100 |
| 79—86 | — | 121 | 150 |

According to the data presented in Table III, the average daily payout of monensin, from a formulation delivered from a device of this invention containing fifty percent by weight of monensin sodium salt, is about 169 mg/head/day for animal #1, 160 mg/head/day for animal #2 and 181 mg/head/day for animal #3, or a mean average daily dose of about 170 mg/head. Such uniform dosing is continuous for about three months. Once all of the formulation contained in the steel capsule has been released, the empty capsule is of such weight that it simply remains in the reticulo-rumen. Additional filled capsules can be administered as needed, and all such capsules can be removed at the time of slaughter. Such removed capsules can be cleaned and refilled with the same or a different formulation and re-administered to ruminant animals, thereby adding economical benefits to the present invention.

In an effort to more fully illustrate particular aspects of this invention, the following detailed examples of the preparation of copolymers and formulations to be delivered by the device of the invention are provided. The examples are representative only and should not be construed as limiting in any respect.

Example 1
Preparation of copolymer matrix

To a 3-neck round bottom flask equipped with a condenser and thermometer were added 355.0 g. of lactic acid, 145.0 g of glycolic acid and 5.0 g of Dowex HCR-W2-H ion exchange resin. The mixture was stirred and heated to 130°C. for three hours, during which time 200 ml. of water were distilled and collected. After discarding the water thus produced, stirring and heating were continued and the pressure was gradually reduced by vacuum over three hours, after which time the temperature of the reactiom mixture had increased to 150°C. at a final pressure of 5 torr. An additional 5.0 g. of Dowex HCR-W2-H catalyst was added to the reaction mixture, and the mixture then was heated to 170°C. at 5.0 torr for twenty-four hours, and then at 185°C. at 5.0 torr for an additional forty-eight hours. The molten reaction mixture was filtered to remove most of the ion exchange polymerization

8

catalyst, and the filtrate was allowed to cool to room temperature to give 300 g. of a copolymer derived from 65 percent lactic acid and 35 percent glycolic acid. The copolymer was analyzed by proton nuclear magnetic resonance spectrometry and shown to consist of 65 percent lactic units and 35 percent glycolic units.

The viscosity of the copolymer was determined in a Ubbelohde viscometer in which chloroform had an efflux time of 51 seconds at 25°C. The copolymer was dissolved in chloroform at a concentration 0.50 g. per 100 ml. of solvent. Inherent viscosity of the copolymer was then determined according to the formulas:

$$\eta r = \frac{t}{t_o}$$

$$\eta inh = \frac{\ln \eta r}{C}$$

wherein:

$\eta r$ = relative viscosity
$t_o$ = efflux time of solvent ($CHCl_3$)
$t$ = efflux time of solution
$\eta inh$ = inherent viscosity
$C$ = conc. in grams/100 ml.
$\ln$ = logarithm

The inherent viscosity of the copolymer was determined to be 0.19 dl/g.

Example 2

Following the general procedure set forth in Example 1, 710 g. of lactic acid and 290 g. of glycolic acid were condensed in the presence of a total of 40.0 g. of Amberlyst 15 ion exchange polymerization catalyst to afford 600 g. of copolymer derived from about 70 percent by weight lactic acid and about 30 percent by weight glycolic acid. The copolymer had the following viscosity: 0.18 dl/g.

Example 3

Following the general procedure of Example 1, 355.0 g. of lactic acid were condensed with 145.0 g. of glycolic acid in the presence of a total of 10.0 g. of Amberlyst 15 ion exchange polymerization catalyst. After removing the catalyst by filtration, there was provided 300 g. of copolymer derived from about 70 percent by weight of lactic acid and 30 percent by weight glycolic acid. The copolymer exhibited the following viscosity: 0.18 dl/g.

Example 4

Following the general procedure of Example 1, 1080 g. of lactic acid were condensed with 252 g. of glycolic acid in the presence of a total of 25.0 g. of Dowex HCR-W2-H ion exchange polymerization catalyst to give, after removal of the catalyst, 750 g. of a copolymer which was shown by NMR to consist of about 79 percent of lactic units and about 21 percent of glycolic units. The copolymer exhibited the following viscosity: 0.20 dl/g.

Example 5

Following the procedure of Example 1, 432 g. of lactic acid were condensed with 101 g. of glycolic acid in the presence of a total of 5.0 g. of Dowex HCR-W2-H ion exchange polymerization catalyst to provide, after work-up, 300 g. of a copolymer derived from about 77 weight percent of lactic acid and about 23 weight percent of glycolic acid. The copolymer had a viscosity of 0.21 dl/g.

Example 6

Following the procedure of Example 1, 432 g. of lactic acid were condensed with 101 g. of glycolic acid in the presence of a total of 2.5 g. of Dowex HCR-W2-H ion exchange polymerization catalyst to provide 300 g. of copolymer derived from about 76 weight percent lactic acid and about 24 weight percent glycolic acid. The copolymer had the following viscosities:

0.12 after 24 hours at 170°C.
0.20 after 24 additional hours at 185°C.
0.23 after 40 additional hours at 185°C.

Example 7

The procedure of Example 1 was followed to condense 1080 g. of lactic acid with 120 g. of glycolic acid in the presence of a total of 25.0 g. of Dowex HCR-W2-H ion exchange polymerization catalyst. After workup, there was recovered 750 g. of a copolymer derived from about 89 weight percent of lactic acid and about 11 weight percent of glycolic acid having the following viscosity: 0.20 dl/g.

Example 8

To a stirred solution of 150 ml. of dichloromethane containing 22.0 g of a copolymer derived from about 80 percent by weight of a lactic acid and about 20 percent by weight of glycolic acid, having an inherent viscosity of about 0.19 dl/g. was added in one portion 22.0 g. of monensin sodium salt. The solution was stirred at ambient temperature for ten minutes and then the solvent was removed by evaporation under reduced pressure. The solid mass that was obtained was ground and heated to about 100°C. and filled into a steel capsule of this invention measuring 35 mm×50 mm and weighing 98.8 g. Final weight of the filled capsule was 142.8 g.

Example 9

To a stirred solution of the copolymer prepared as described in Example 7 in a solvent such as chloroform is added salinomycin, and diluents such as beeswax and sorbitan monostearate. The solution is stirred at 25°C. for several minutes and then the solvent is removed by evaporation under reduced pressure. The residue is dissolved in fresh chloroform and spray dried by conventional means to provide a controlled release formulation of salinomycin ideally suited for filling into a capsule delivery device of this invention.

Example 10

A formulation comprised of about 5.0 g. of monensin sodium salt in about 7.0 g. of a copolymer derived from about 60 weight percent lactic acid and about 40 weight percent glycolic acid, having an inherent viscosity of about 0.20 dl/g., is extruded into rods and then melted at 100°C. and filled into a steel cylinder measuring about 10 mm in diameter and about 20 mm in length, said steel cylinder being open at both ends. The bolus thus prepared is orally administered to a sheep weighing about 70 pounds for the effective promotion of growth over a four month period.

**Claims**

1. A drug delivery device for oral delivery to the reticulo-rumen of a ruminant a controlled release formulation, said device comprising a steel cylinder open at both ends, said cylinder having an inside diameter to length ratio of 0.50 to 0.75, having a density from 2.0 to 3.5 g/cc, formulation retaining means being provided on or in engagement with the inner wall of the cylinder, said device being coated with a substance which does not promote depolymerization of a copolymer derived from 60 to 95 weight percent lactic acid and 40 to 5 weight percent glycolic acid.

2. The device of claim 1 wherein the inside diameter to length ratio is 0.55 to 0.75.

3. The device of claim 1 or claim 2 wherein the retaining means is a multiplicity of grooves cut in the innersurface of the walls, said grooves having a depth of 20 to 50 percent of the wall thickness.

4. The device of claim 3 wherein said inner grooves are 0.2 to 2.0 millimeters in width and 1 to 5 millimeters apart.

5. The device of claim 4 wherein said inner grooves are 0.6 to 1.0 millimeters in width and 2 to 3 millimeters apart.

6. The device of claim 5, said device having an inside diameter of 11 to 35 millimeters and a length of 20 to 64 millimeters.

7. The device of claim 6, said device measuring 30 millimeters in diameter and 50 millimeters in length.

8. The device of claim 6, said device measuring 15 millimeters in diameter and 25 millimeters in length.

9. The device of claim 1 or claim 2 wherein said coating comprises nickel, magnesium, silver or aluminum.

10. The device of claim 9 wherein said coating comprises nickel.

11. The device of claim 1 or claim 2 wherein said coating comprises a food grade enamel.

12. The device of claim 11 wherein said coating comprises epoxyphenolic resins.

13. The device of claim 1 or claim 2 wherein said density is 2.9 g/cc.

14. A drug delivery device according to any preceding claim, charged with a sustained release formulation of an active ingredient exhibiting a desired effect on ruminant animals.

**Revendications**

1. Dispositif distributeur de médicament pour la distribution, par voie orale, dans le réticulo-rumen d'in ruminant, d'une formulation à libération réglée, ce dispositif comprenant un cylindre en acier ouvert à ses deux extrémités, ce cylindre ayant un rapport diamètre intérieur/longueur de 0,50 à 0,75 et une densité de 2 à 3,5 g/cm³, un moyen de retenue de formulation étant prévu sur ou engagé dans la paroi intérieure du cylindre, ce dispositif étant enduit d'une substance qui ne favorise pas la dépolymérisation d'un copolymère dérivant de 60 à 95% en poids d'acide lactique et de 40 á 5% en poids d'acide glycolique.

2. Dispositif suivant la revendication 1, caractérisé en ce que la rapport diamètre intérieur/longueur est de 0,55 à 0,75.

3. Dispositif suivant la revendication 1 ou 2, caractérisé en ce que le moyen de retenue est constitué de plusieurs gorges pratiquées dans la surface intérieure des parois, ces gorges ayant une profondeur représentant 20 à 50% de l'épaisseur de paroi.

4. Dispositif suivant la revendication 3, caractérisé en ce que ces gorges internes ont une largeur de 0,2 à 2 mm et sont espacées l'une de l'autre d'une distance de 1 à 5 mm.

5. Dispositif suivant la revendication 4, caractérisé en ce que ces gorges internes ont une largeur de 0,6 à 1 mm et sont espacées l'une de l'autre d'une distance de 2 à 3 mm.

6. Dispositif suivant la revendication 5, ce dispositif ayant un diamètre intérieur de 11 à 35 mm et une longueur de 20 à 64 mm.

7. Dispositif suivant la revendication 6, ce dispositif ayant un diamètre de 30 mm et une longueur de 50 mm.

8. Dispositif suivant la revendication 6, ce dispositif ayant un diamètre de 15 mm et une longueur de 25 mm.

9. Dispositif suivant la revendication 1 ou 2, ce revêtement étant constitué de nickel, de magnésium, d'argent ou d'aluminium.

10. Dispositif suivant la revendication 9, caractérisé en ce que ce revêtement est constitué de nickel.

11. Dispositif suivant la revendication 1 ou 2, caractérisé en ce que le revêtement est constitué d'un émail de qualité pour produits alimentaires.

12. Dispositif suivant la revendication 11, caractérisé en ce que ce revêtement est constitué de résines époxy-phénoliques.

13. Dispositif suivant la revendication 1 ou 2, caractérisé en ce que cette densité est de 2,9 g/cm$^3$.

14. Dispositif distributeur de médicament suivant l'une quelconque des revendications précédentes, ce dispositif étant chargé d'une formulation à libération prolongée comprenant un ingrédient actif exerçant un effet souhaité chez les ruminants.

## Patentansprüche

1. Wirkstoff liefernde Forrichtung für das orale einführen in den zweiten Pansen (reticulo-rumen) eines Wiederkäuers zwecks dosierter Verabreichung des Wirkstoffes, dadurch gekennzeichnet, daß die Vorrichtung einen an beiden Enden offenen Stahlzylinder umfaßt, dessen Verhältnis von Innendurchmesser zur Länge 0,50 bis 0,75 und dessen Dicht 2,0 bis 3,5 g/cm$^3$ beträgt, der an seiner Innenmantelfläche mit Wirkstoffaufnahmemitteln versehen ist und der einen Überzug aus einem Material aufweist, das keine Beschleunigung der Depolymerisation eines aus 60 dis 95 Gewichtsprozent Milchsäure und 40 bis 5 Gewichtsprozent Glykolsäure bestehenden Mischpolymerisats bewirkt.

2. Vorrichtung nach Anspruch 1, dadurch gekennzeichnet, daß das Verhältnis von Innendurchmesser zur Länge 0,55 bis 0,75 beträgt.

3. Vorrichtung nach Anspruch 1 oder Anspruch 2, dadurch gekennzeichnet, daß die Wirkstoffaufnahmemittel aus einer Vielzahl von in die Innenmantelfläche geschnittener Nuten bestehen, die eine Tiefe von 20% bis 50% der Wandstärke aufweisen.

4. Vorrichtung nach Anspruch 3, dadurch gekennzeichnet, daß Nuten 0,2 bis 2,0 mm breit und 1 bis 5 mm voneinander entfernt angeordnet sind.

5. Vorrichtung nach Anspruch 4, dadurch gekennzeichnet, daß die Nuten 0,6 bis 1,0 mm breit und 2 bis 3 mm voneinander entfernt angeordnet sind.

6. Vorrichtung nach Anspruch 5, dadurch gekennzeichnet, daß ihr Innendurchmesser 11 bis 35 mm und ihre Länge 20 bis 64 mm beträgt.

7. Vorrichtung nach Anspruch 6, dadurch gekennzeichnet, daß sie einen Gesamtdurchmesser von 30 mm und eine Länge von 50 mm aufweist.

8. Vorrichtung nach Anspruch 6, dadurch gekennzeichnet, daß sie einen Gesamtdurchmesser von 15 mm und eine Länge von 25 mm aufweist.

9. Vorrichtung nach Anspruch 1 oder Anspruch 2, dadurch gekennzeichnet, daß ihr Überzug Nickel, Magnesium, Silber oder Aluminium enthält.

10. Vorrichtung nach Anspruch 9, dadurch gekennzeichnet, daß ihr Überzug Nickel enthält.

11. Vorrichtung nach Anspruch 1 oder Anspruch 2, dadurch gekennzeichnet, daß ihr Überzug ein den Futtermittelvorschriften entsprechendes Email enthält.

12. Vorrichtung nach Anspruch 11, dadurch gekennzeichnet, daß ihr Überzug Epoxyphenolharz enthält.

13. Vorrichtung nach Anspruch 1 oder Anspruch 2, dadurch gekennzeichnet, daß ihre Dichte 2,9 g/cm$^3$ beträgt.

14. Wirkstoff liefernde Vorrichtung nach einem der vorhergehenden Ansprüche, gekennzeichnet durch eine Füllung zur kontinuierlichen Freigabe eines aktiven Bestandteils zwecks Einleitung eines gewünschten Effektes bei Wiederkäuern.

Fig. 1

Fig. 2

Fig. 3